# EUROPEAN PATENT SPECIFICATION

(11) **EP 1 812 059 B1**
(45) Date of publication and mention of the grant of the patent: **19.05.2010**
(21) Application number: 05800771.7
(22) Date of filing: 08.11.2005
(51) Int. Cl.: A61K 39/35

(54) **METHOD OF PREVENTIVE TREATMENT OF ALLERGY BY MUCOSAL ADMINISTRATION OF AN ALLERGY VACCINE**
VERFAHREN FÜR DIE PRÄVENTIVE BEHANDLUNG VON ALLERGIEN DURCH VERABREICHUNG EINER ALLERGIE-VAKZINE AUF DIE SCHLEIMHÄUTE
METHODE DE TRAITEMENT PREVENTIF CONTRE LES ALLERGIES PAR ADMINISTRATION PAR LES MUQUEUSES D'UN VACCIN CORRESPONDANT

(30) Priority: 10.11.2004 DK 200401730; 10.11.2004 US 626454 P; 03.06.2005 US 686914 P
(43) Date of publication of application: 01.08.2007
(73) Proprietor: Alk-Abelló A/S, 2970 Hørsholm (DK)
(72) Inventor: BRIMNES, Jens, 2730 Herlev (DK); KILDSGAARD, Jens, 2840 Holte (DK)
(74) Representative: Inspicos A/S
(86) International application number: PCT/DK2005/000716
(87) International publication number: WO 2006/050729

(56) References cited:
- WO-A-03/096869
- WO-A-2004/047793
- US-B1- 6 333 038
- FANTA C ET AL: "SYSTEMIC IMMUNOLOGICAL CHANGES INDUCED BY ADMINISTRATION OF GRASS POLLEN ALLERGENS VIA THE ORAL MUCOSA DURING SUBLINGUAL IMMUNOTHERAPY" INTERNATIONAL ARCHIVES OF ALLERGY AND IMMUNOLOGY, vol. 120, no. 3, November 1999 (1999-11), pages 218-224, XP008044918 ISSN: 1018-2438
- LIMA M TORRES ET AL: "Grass pollen sublingual immunotherapy for seasonal rhinoconjunctivitis: a randomized controlled trial." CLINICAL AND EXPERIMENTAL ALLERGY : JOURNAL OF THE BRITISH SOCIETY FOR ALLERGY AND CLINICAL IMMUNOLOGY. APR 2002, vol. 32, no. 4, April 2002 (2002-04), pages 507-514, XP002334115 ISSN: 0954-7894
- BUFE A ET AL: "Efficacy of sublingual swallow immunotherapy in children with severe grass pollen allergic symptoms: a double-blind placebo-controlled study." ALLERGY. MAY 2004, vol. 59, no. 5, May 2004 (2004-05), pages 498-504, XP002334116 ISSN: 0105-4538
- HOLT P G ET AL: "SUBLINGUAL ALLERGEN ADMINISTRATION. I. SELECTIVE SUPPRESSION OF IGE PRODUCTION IN RATS BY HIGH ALLERGEN DOSES" CLINICAL ALLERGY, BLACKWELL SCIENTIFIC PUBLICATIONS, OXFORD, GB, vol. 18, no. 3, May 1988 (1988-05), pages 229-234, XP008047914 ISSN: 0009-9090 cited in the application
- WATANABE TOHRU ET AL: "Roles of FcgammaRIIB in nasal eosinophilia and IgE production in murine allergic rhinitis." AMERICAN JOURNAL OF RESPIRATORY AND CRITICAL CARE MEDICINE. 1 JAN 2004, vol. 169, no. 1, 1 January 2004 (2004-01-01), pages 105-112, XP002385536 ISSN: 1073-449X
- CIRLA A M ET AL: "A pre-seasonal birch/hazel sublingual immunotherapy can improve the outcome of grass pollen injective treatment in bisensitized individuals. A case-referent, two-year controlled study" ALLERGOLOGIA ET IMMUNOPATHOLOGIA, GARSI, MADRID,, ES, vol. 31, no. 1, January 2003 (2003-01), pages 31-43, XP002322832 ISSN: 0301-0546
- VRTALA S ET AL: "Strategies for converting allergens into hypoallergenic vaccine candidates" METHODS : A COMPANION TO METHODS IN ENZYMOLOGY, ACADEMIC PRESS INC., NEW YORK, NY, US, vol. 32, no. 3, March 2004 (2004-03), pages 313-320, XP004488982 ISSN: 1046-2023

## Description

### TECHNICAL FIELD

The present invention relates to a method of preventive treatment of allergy to an allergen in a subject comprising administering an allergy vaccine to a mucosal surface of the subject.

### BACKGROUND OF THE INVENTION

Allergy is a major health problem in countries where Western lifestyle is adapted. Furthermore, the prevalence of allergic disease is increasing in these countries. Although allergy in general may not be considered a life-threatening disease, asthma annually causes a significant number of deaths. An exceptional prevalence of about 30% in teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. Firstly, a progression of symptoms and disease severity, as well as disease progression, for example from hay fever to asthma.

Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen sources are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. It therefore follows that a foreign substance having only one antibody binding epitope does not initiate an allergic reaction. The cross-linking of receptor bound IgE on the surface of mast-cells also leads to release of signaling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with by the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, neither do they control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Specific allergy vaccination is, in spite of its virtues, not in widespread use, primarily for two reasons. One reason is the inconveniences associated with the traditional vaccination programme that comprises repeated vaccinations i.a. injections over a several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

Attempts to improve vaccines for specific allergy vaccination have been performed for over 30 years and include multifarious approaches. Several approaches have addressed the allergen itself through modification of the IgE reactivity.

Fanta et al. (Int. Arch. Allergy Immunol, 1999, 120: 218-224) relates to a study of the immunological changes induced by SLIT in group of grass pollen allergic patients selected according to the criteria of having clinical symptoms (rhinitis and/or seasonal bronchial asthma) during the grass pollen season, a positive skin prick test to grass pollen extracts, and specific IgE to grass pollen as determined by RAST-CAP. The SLIT was carried out by sublingual administration of drops of allergen extract.

Holt et al. ("Suppression of IgE responses following inhalation of antigen", Immunology Today, vol. 8, No. 1, 1987) mentions the fact that as a response to exposure to inhaled or oronasallly instilled allergen, tolerance is induced in the upper respiratory tract corresponding to that induced in the gastrointestinal tract by dietary antigens.

Holt et al. ("Sublingual allergen administration. I. Selective suppression of IgE production in rats by high allergen doses", Clinical Allergy, 1988, Volume 18, pages 229-234) relates to sublingual administration of an allergen (ovalbumin) to naïve rats for seven consecutive days followed by a parenteral challenge by ovalbumin five days after the last sublingual dose. The results showed a selective suppression of IgE specific to ovalbumin. It is speculated that the mechanism of the treatment involves stimulation of allergen-specific suppressor cells. It is further mentioned that the treatment proposed involves naïve animals and should be distinguished from a sublingual desensitisation process.

WO 95/17208 discloses a method of prevention of allergic disease comprising administering to a previously unsensitised subject a dose of allergen effective to induce establishment of a stable population of allergen-specific T-helper-1-like memory lymphocytes capable of inhibiting activity of allergen-specific T-helper-2-like lymphocytes. The subject to be treated is preferably between 3 months and 7 years. As allergen e.g. house dust mites, grass pollen and tree pollen are mentioned. The administration of the allergen may be carried out by the oral, intranasal, oronasal, rectal, intradermal, intramuscular or subcutaneous route.

The home page www.immunetolerance.org (11 October 2004) discloses e.g. a planned clinical study of preventive treatment of children without sensitisation to inhalants, wherein sublingual drops containing either allergen (house dust mite, timothy grass and cat) are administered to the children, and wherein the children are followed for the development of allergy for three years. The children recruited for the study have a history of atopic dermatitis or food allergy and their biological mother or father or one sibling has a history of atopy.

US 6,333,038 discloses regulation of development of normal anti-allergen immunity by administering allergen to children, which have not been sensitised.

The object of the present invention is to provide an improved method of preventive treatment of individuals, in particular children.

### SUMMARY OF THE INVENTION

This object is obtained with the present invention, which relates to an allergen for use in preventive treatment of allergy by administering an allergy vaccine containing the allergen to a mucosal surface of the subject,
a) wherein the subject to be treated is sensitised so as to exhibit an IgE response specific to the allergen,
b) wherein the subject to be treated is free of clinical symptoms of the allergy associated with the allergen, and
c) wherein the preventive treatment is aimed at preventing or reducing subsequent clinical symptoms of the allergy associated with the allergen.

The invention is based on the novel finding that it is possible to prevent symptoms of allergy to an allergen from developing in an individual, whose immune system has been exposed to the allergen, but wherein the immune response has not yet progressed into a state involving clinical symptoms, such as rhinitis, conjunctivitis, rhinorrhea, nasal obstruction, sinusitis, sneezing, atopic dermatitis, itching, watery eyes, watery nose, wheezing and skin irritation. It has hitherto been believed that in order to achieve an effective preventive treatment of an allergy the individual to be treated should be unsensitised. Also, it has hitherto been believed that the mechanism involved in prevention of an allergy is induction of oral tolerance corresponding to that induced in the gastrointestinal tract by dietary antigens. However, in accordance with the present invention it has surprisingly been shown that it is possible to achieve a preventive effect by treating individuals, who are already sensitised and who do not yet show any clinical symptoms. It is believed that the preventive effect obtained takes effect through a mechanism, which is partly or wholly similar to the mechanism of desensitisation taking place in specific allergy vaccination (immunotherapy).

It is further believed that the preventive treatment is most effective when carried out as soon after sensitisation as possible before the immune system response begins to shift further toward an allergic Th2 cell response. In other words it is in general advantageous to treat children as young as possible once they have been exposed to an allergen. Also, it is believed that due to the effectiveness of such early preventive treatment, treatment may be effected with smaller doses, fewer administrations and/or a shorter period of treatment compared to specific allergy vaccination of adults with developed clinical symptoms. Due to the mildness of the protocol of the preventive treatment, it is suitable for use in general vaccination programs of all children or large groups of selected children.

A further advantage of the invention is that sensitised persons are the persons most likely to develop allergy, and hence sensitised persons constitute the most relevant group of persons for subjecting to preventive treatment.

The invention further relates to the use of an allergen for the manufacture of an allergy vaccine comprising the allergen as acitve substance for preventive treatment of allergy by administering the allergy vaccine to a mucosal surface of a subject,
a) wherein the subject to be treated is sensitised so as to exhibit an IgE response specific to the allergen,
b) wherein the subject to be treated is free of clinical symptoms of the allergy associated with the allergen, and
c) wherein the preventive treatment is aimed at preventing or reducing subsequent clinical symptoms of the allergy associated with the allergen.

### SHORT DESCRIPTION OF THE FIGURES

Fig. 1A shows clinical data (number of sneezes) in sensitized mice challenged intranasally with either 5000 SQ Phl p or Buffer.
Fig. 1B shows the serum IgE level in sensitized mice challenged intranasally with either 5000 SQ Phl p or Buffer.
Fig. 1C shows the BAL IgE level in sensitized mice challenged intranasally with either 5000 SQ Phl p or Buffer.
Fig. 1 D shows the NAL IgE level in sensitized mice challenged intranasally with either 5000 SQ Phl p or Buffer.
Fig. 1 E shows the NAL eosinophil level in sensitized mice challenged intranasally with either 5000 SQ Phl p or Buffer.
Fig. 2A shows clinical data (number of sneezes) in sensitized mice subjected to either SLIT or buffer treatment and challenged intranasally with Phl p.
Fig. 2B shows airway hyperresponsiveness in response to metacholine challenge (as measured by Penh values) in sensitized mice subjected to either SLIT or buffer treatment and challenged intranasally with Phl p.
Fig. 3A shows the serum IgE level in sensitized mice subjected to either SLIT or buffer treatment and challenged intranasally with Phl p.
Fig. 3B shows the serum IgG₁ level in sensitized mice subjected to either SLIT or buffer treatment and challenged intranasally with Phl p.
Fig. 4A shows the BAL IgE level in sensitized mice subjected to either SLIT or buffer treatment and challenged intranasally with Phl p.
Fig. 4B shows the NAL IgE level in sensitized mice subjected to either SLIT or buffer treatment and challenged intranasally with Phl p.
Fig. 4C shows the BAL IgA level in sensitized mice subjected to either SLIT or buffer treatment and challenged intranasally with Phl p.
Fig. 4D shows the NAL IgA level in sensitized mice subjected to either SLIT or buffer treatment and challenged intranasally with Phl p.
Fig. 5A shows the eosinophil peroxidase level in BAL in sensitized mice subjected to either SLIT or buffer treatment and challenged intranasally with Phl p.
Fig. 5B shows the eosinophil peroxidase level in NAL in sensitized mice subjected to either SLIT or buffer treatment and challenged intranasally with Phl p.
Fig. 6A shows the T cell proliferation in splen in sensitized mice subjected to either SLIT or buffer treatment and challenged intranasally with Phl p.
Fig. 6B shows the T cell proliferation in lymph nodes (LN) cells in sensitized mice subjected to either SLIT or buffer treatment and challenged intranasally with Phl p.

### DETAILED DESCRIPTION OF THE INVENTION

### Allergen

The allergen according to the present invention may be any naturally occurring protein that has been reported to induce allergic, i.e. IgE mediated, reactions upon their repeated exposure to an individual.
Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, gasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are i.a. such originating from the genera Alternaria and Cladosporium.

In a particular embodiment of the invention the allergen is Bet v 1, Aln g 1, Cor a 1 and Car b 1, Que a 1, Cry j 1, Cry j 2 , Cup a 1, Cup s 1, Jun a 1, Jun a 2, jun a 3, Ole e 1 , Lig v 1, Pla I 1, Pla a 2, Amb a 1, Amb a 2, Amb t 5, Art v 1, Art v 2 Par j 1 , Par j 2, Par j 3, Sal k 1, Ave e 1, Cyn d 1, Cyn d 7, Dac g 1, Fes p 1, Hol l 1, Lol p 1 and 5, Pha a 1, Pas n 1, Phl p 1, Phl p 5, Phl p 6, Poa p 1, Poa p 5, Sec c 1, Sec c 5, Sor h 1, Der f 1 , Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2 , Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding of any of these.

In a preferred embodiment of the invention the allergen is selected from the group consisting of a tree pollen allergen, a grass pollen allergen, a dust mite allergen, a herb allergen and an animal allergen. Preferably, the allergen is selected from the group consisting of a grass pollen allergen, a dust mite allergen, a ragweed allergen, a cedar pollen, a cat allergen and a birch allergen.

In yet another embodiment of the invention the formulation comprises at least two different types of allergens either originating from the same allergic source or originating from different allergenic sources e.g. grass group 1 and grass group 5 allergens or mite group 1 and group 2 allergens from different mite and grass species respectively, weed antigens like short and giant ragweed allergens, different fungis allergens like alternaria and cladosporium, tree allergens like birch, hazel, hornbeam, oak and alder allergens, food allergens like peanut, soybean and milk allergens.

The allergen incorporated into the formulation may be in the form of an extract, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. In another preferred embodiment the allergen is a recombinant allergen. In a further preferred embodiment the allergen is a naturally occurring low IgE-binding mutant or a recombinant low IgE-binding mutant.

Allergens may be present in equi-molar amounts or the ratio of the allergens present may vary preferably up to 1:20.

In a further embodiment of the invention the low IgE binding allergen is an allergen according to WO 99/47680, WO 02/40676 or WO 03/096869 A2.

### Preventive treatment

Specific allergy vaccination (SAV), formerly known as Specific Immunotheraphy or Hyposensitization, has been used for the treatment of Type 1 IgE mediated allergic disease since the beginning of this century.

The general benefits obtained through SAV are: a) reduction of allergic symptoms and medicine consumption, b) improved tolerance towards the allergens in the eyes, nose and lungs and c) reduced skin reactivity (early and late phase reactions).

The basic mechanism behind the improvement obtained by SAV is unknown, but a number of common features can be extracted from the numerous SAV studies performed in the last decades: 1) the amount of total IgE is unchanged during the treatment period, 2) the amount of allergen specific IgE increases transiently during updosing, then it falls back to the initial (pretreatment) level, 3) the epitope specificity and affinity of IgE remains ' unchanged, 4) allergen specific IgG, in particularly IgG4, raises sharply during SAV, 5) a new Th0/1/Reg response is apparently initiated and 6) the Th2 response seem unchanged. There is no correlation between the effect induced by SAV and the onset of specific IgG.

SAV induces a new immune response which matures during the treatment period (Th0/1 T-cells are recruited, an allergen specific IgX (X may be A1, A2, G1, G2, G3, G4, M or D) is initiated). As the affinity (or amount/affinity) of the new antibody response, IgX, has matured, IgX may compete efficiently with IgE for the allergen(s), inhibiting the "normal" Th2 based allergic response characterised by the cross-linking of receptor bound IgE on the surface of mast-cells and basophils. Hence, clinical symptoms will gradually be reduced.

It is believed that the preventive treatment carried out in the present invention at least partly functions by way of the same mechanisms as disclosed above for SAV.

The mucosa to which the allergy vaccine is administered may be any suitable mucosa, and the administration includes oral (via the mucosa of the digestive system), nasal, vaginal, sublingual, ocular, rectal, urinal, intramammal, pulmonal, otolar (i.e. via the ear) and buccal administration, preferably buccal or sublingual administration (oromucosal administration). The allergy vaccine may be in the form of a spray, an aerosol, a mixture, a suspension, a dispersion, an emulsion, a gel, a paste, a syrup, a cream, an ointment, implants (ear, eye, skin, nose, rectal, and vaginal), intramammary preparations, vagitories, suppositories, or uteritories.

It has been speculated that it is preferable to carry out a mucosal administration of a vaccine via the mucosa, which is subject to the natural exposure to the antigenic agent. Accordingly, for allergies to airborne mucosal antigenic agents, it is preferred to use administration via the respiratory system, preferably an oromucosal administration.
Correspondingly, for allergies to mucosal antigenic agents, which comes into contact with the mucosa of the digestive system, it is preferred to use oral administration.

In one embodiment of the invention, the subject is subjected to a vaccination protocol comprising daily administration of the vaccine. In another embodiment of the invention the vaccination protocol comprises administration of the vaccine every second day, every third day or every fourth day. For instance, the vaccination protocol comprises administration of the vaccine for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non-administration is shorter than the (total) period of administration.

In a further embodiment of the invention, the vaccine is administered to the test individual once a day. Alternatively, the vaccine is administered to the test individual twice a day. The vaccine may be a uni-dose vaccine.

### Oromucosal administration

The oromucosal administration may be carried out using any available oromucosal administration formulation, including a solution, a suspension, fast dispersing dosage forms, drops and lozenges.

In a preferred embodiment of the invention, sublingual immunotherapy (SLIT) is used, in which case fast dispersing dosage forms, drops and lozenges are preferred formulations.

Examples of fast dispersing dosage forms are those disclosed in US-A-5,648,093, WO 00/51568, WO 02/13858, WO99/21579, WO 00/44351, US-A-4,371,516 and EP-278 877, as well as co-pending DK PA 2003 00279 and DK PA 2003 00318 filed in the assignee name of ALK-Abelló A/S. Preferred fast dispersing dosage forms are those produced by freeze-drying. Preferred matrix forming agents are fish gelatine and modified starch.

Classical incremental dosage desensitisation, where the dose of allergen in the form of a fast dispersing solid dosage form is increased to a certain maximum, may be used in the present invention. The preferred potency of a unit dose of the dosage form is from 150 - 1000000 SQ-u/dosage form, more preferred the potency is from 500 - 500000 SQ-u/dosage form and more preferably the potency is from 1000 - 250000 SQ-u/dosage form, even more preferred 1500-125000 SQ-u/dosage form most preferable 1500-75000 SQ-u/dosage form.

In another embodiment of the invention the dosage form is a repeated monodose, preferably within the range of 1500-75000 SQ-u/dosage form.

### Sensitisation

The subject to be treated is sensitised so as to exhibit an IgE response specific to the allergen administered. In connection with the present invention the expression "exhibit an IgE response specific to the allergen" means a level of allergen-specific IgE antibody detectable in at least one immunoassay. The detection of the allergen-specific IgE antibody may be carried out using any conventional immunoassay, e.g. those described in WO 94/11734 and WO 99/67642.

In a particular embodiment of the invention the subject is further sensitised to exhibit a Th2 cell response specific to the allergen.

In a particular embodiment of the invention the subject is further sensitised to exhibit a positive allergen-specific response in a Skin Prick Test (SPT).

In a further particular embodiment of the invention the subject is less than 40 years, preferably less than 30 years, more preferably less than 20 years and most preferably between 2 and 10 years of age.

### Clinical symptoms

The subject to be treated is free of clinical symptoms of the allergy associated with the allergen.

The clinical symptoms of the allergy associated with the allergen may be any conventional symptom, including rhinitis, conjunctivitis, rhinorrhea, nasal obstruction, sinusitis, sneezing, atopic dermatitis, itching, watery eyes, watery nose, wheezing and skin irritation.

A number of factors are indicative for development of allergy with manifested clinical symptoms later in life. In the following subjects exhibiting one or more such indicating factors are referred to as high risk subjects. Indicating factors of high risk subjects are clinical symptoms of allergies associated with one or more allergens other than the allergen of the vaccine. Further indicating factors of high risk subjects are the presence of one or more allergies in one or both parents or grandparents or in one or more sibling. The preventive treatment according to the invention is particularly suitable for high risk subjects. However, the subject to be treated may also be a subject exhibiting no indicating factors of high risk subjects, e.g. free of clinical symptoms of allergy to other allergens.

### Formulation of allergy vaccine

The allergy vaccine used in the invention may be in the form of any formulation suitable for administration to a mucosal surface, including a spray, an aerosol, a mixture, tablets (entero- and not-enterocoated), capsule (hard and soft, entero- and not-enterocoated), a suspension, a dispersion, granules, a powder, a solution, an emulsion, chewable tablets, drops, a gel, a paste, a syrup, a cream, a losenge (powder, granulate, tablets), a fast-dispersing tablet, an instillation fluid, a gas, a vapour, an ointment, a stick, implants (ear, eye, skin, nose, rectal, and vaginal), intramammary preparations, vagitories, suppositories, or uteritories.

It is to be understood that the vaccine of the invention may further comprise additional adjuvants and other excipients suitable for such type of formulation. Such additional adjuvants and excipients are well-known to the person skilled in the art and include i.a. solvents, emulsifiers, wetting agents, plasticizers, colouring substances, fillers, preservatives, viscosity adjusting agents, buffering agents, mucoadhesive substances, and the like. Examples of formulation strategies are well-known to the person skilled in the art.

### Adjuvant

The mucosal allergy vaccine may include an adjuvant, which may be any conventional adjuvant, including oxygen-containing metal salts, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1β, IL-2, IL-7, IL-12, INFγ), GM-CSF, MDF derivatives, CpG oligonucleotides, LPS, MPL, phosphophazenes, Adju-Phos®, glucan, antigen formulation, liposomes, DDE, DHEA, DMPC, DMPG, DOC/Alum Complex, Freund's incomplete adjuvant, ISCOMs®, LT Oral Adjuvant, muramyl dipeptide, monophosphoryl lipid A, muramyl tripeptide, and phospatidylethanolamine.

The oxygen-containing metal salt may be any oxygen-containing metal salt providing the desired effect. In a preferred embodiment, the cation of the oxygen-containing metal salt is selected from Al, K, Ca, Mg, Zn, Ba, Na, Li, B, Be, Fe, Si, Co, Cu, Ni, Ag, Au, and Cr. In a preferred embodiment, the anion of the oxygen-containing metal salt is selected from sulphates, hydroxides, phosphates, nitrates, iodates, bromates, carbonates, hydrates, acetates, citrates, oxalates, and tartrates, and mixed forms thereof.
Examples are aluminium hydroxide, aluminium phosphate, aluminium sulphate, potassium aluminium sulphate, calcium phosphate, Maalox (mixture of aluminium hydroxide and magnesium hydroxide), beryllium hydroxide, zinc hydroxide, zinc carbonate, zinc chloride, and barium sulphate.

Allergy vaccines in the form of an aqueous solution or a fast-dispersing tablet, cf. WO 04/047794, are particularly suitable for buccal and sublingual administration.

### DEFINITIONS

In connection with the present invention the following definitions are used:
The term "oromucosal administration" refers to a route of administration where the dosage form is placed under the tongue or anywhere else in the oral cavity (buccal administration) to allow the active ingredient to come in contact with the mucosa of the oral cavity or the pharynx of the patient in order to obtain a local or systemic effect of the active ingredient. An example of an oromucosal administration route is sublingual administration.
The term "sublingual administration" refers to a route of administration, where a dosage form is placed underneath the tongue in order to obtain a local or systemic effect of the active ingredient.
The term "SQ-u" means SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.
The expression "immunomodulating treatment" means that the treatment modulates the immune response of the subject treated.

### EXAMPLES

### EXAMPLE 1: The effect of SLIT in a mouse model of Rhinitis

### Rationale:

The rhinitis model was set up in order to test the effect of sublingual immunotherapy (SLIT) in a mouse model with clinical manifestations.

### Methods:

### Animals

Female, 6-10 week-old BALB/c mice were bred in-house and maintained on a defined diet not containing components cross reacting with antisera to Phleum pratense (Phl p). Each experimental group consisted of 8 - 10 animals.

### Animal experiments

Mice were sensitized by three intraperitoneal (ip) injections of Phl p extract adsorbed to alum, followed by sublingual treatment with Phl p-extract or buffer for 6-9 weeks. The mice were subsequently challenged intranasally for two weeks with Phl p-extract and analyzed for clinical symptoms as described below. Following sacrifice blood, bronchoalveolar fluid (BAL), nasopharyngeal fluid (NAL), spleen and cervical lymph nodes were collected for analysis.

### Clinical data

Sneezing and nose rubbing: The mice were observed in a 10 min-period after intranasal administration of Phl and the number of sneezes and nose-rubbings were counted.

Airway hyperreactivity: Using a whole body pletysmograph airflow obstruction was induced by increasing concentrations of aerosolized metacholine. Pulmonary airflow obstruction was measured by enhanced pause (penh) in a period of 6 minutes after administration of metacholine.

### IgA assay

Estapore magnetic beads (Estapore IB-MR/0,86) coupled to goat anti-mouse IgA are incubated with BAL or NAL. Then washing and incubation with biotinylated allergen is carried out. Then washing and incubation with streptavidin labeled LITE reagent is carried out, and after washing light luminescence is measured in a luminometer (Magic Lite Analyser EQ).

### IgE assay

Estapore magnetic beads (Estapore IB-MR/0,86; A0201 ) coupled to anti-mouse IgE are incubated with mouse serum. Then washing and incubation with biotinylated allergen is carried out. Then washing and incubation with streptavidin labeled LITE reagent is carried out, and after washing light luminescence is measured in a luminometer (Magic Lite Analyser EQ).

### IgG. IgG1 and IgG2a assay

Phl p (10 µg/ml) extract is added to the wells of an ELISA plate and the plates are allowed to stand until the next day at 4-8 °C. Then the coated plates are washed with a buffer and blocked with 2 % Casein buffer for one hour at room temperature on a shaking table. After removing the casein buffer, the diluted serum sample is added to the plate and incubated at room temperature for two hours on a shaking table. The plates are washed and biotinylated rabbit anti-mouse IgG/IgG1/IgG2a diluted in 0.5 % BSA buffer is added to each well and allowed to stand at room temperature for one hour on a shaking table. After washing, streptavidin-HRP diluted in 0.5 % BSA buffer is added to each well and allowed to stand at room temperature for one hour on a shaking table. The plates are developed with TMP substrate (3,3',5,5'-Tetramethylbenzidine) for 20 min and stopped with 0.5 M H2SO4. The resulting reaction mixtures are subjected to a spectrophotometric measurement at 450 nm endpoint.

### T-cell proliferation assay

Spleens were teased into single cell suspension and washed three times in medium. Cells were counted and adjusted to 1.67 x 106 cells/mL. 3 x 105 cells were added to each well of a 96 well flat-bottomed culture plate and the cells were stimulated by 0, 10 and 40 µg/mL Phl p extract. The cells were cultured for 6 days at 37 °C and 5% CO2. Proliferation was measured by adding 0.5 µCi of 3H-thymidine to each well for the last 18 hours of the culture period, followed by harvesting the cells and counting the incorporated radiolabel.

### Results

### Induction of Rhinitis

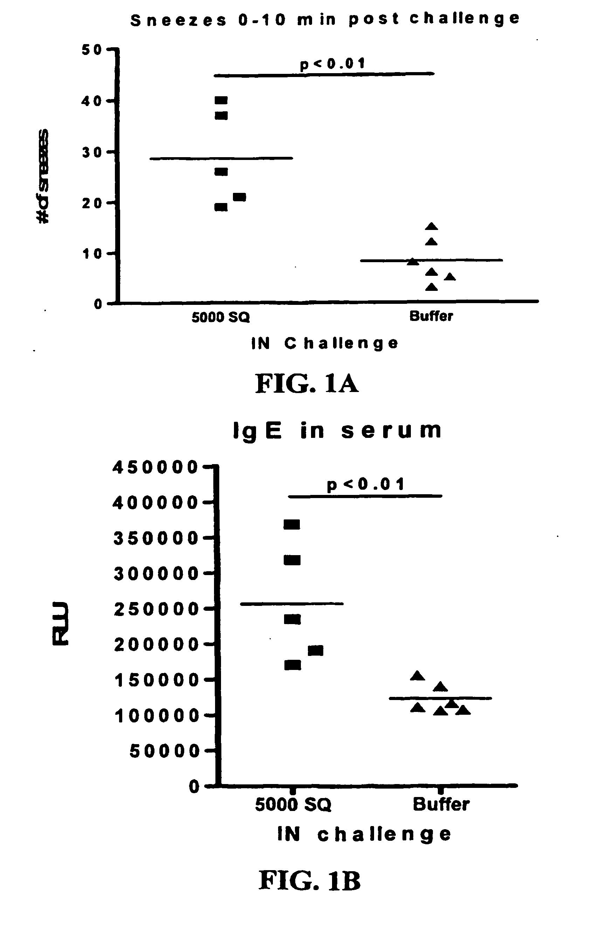

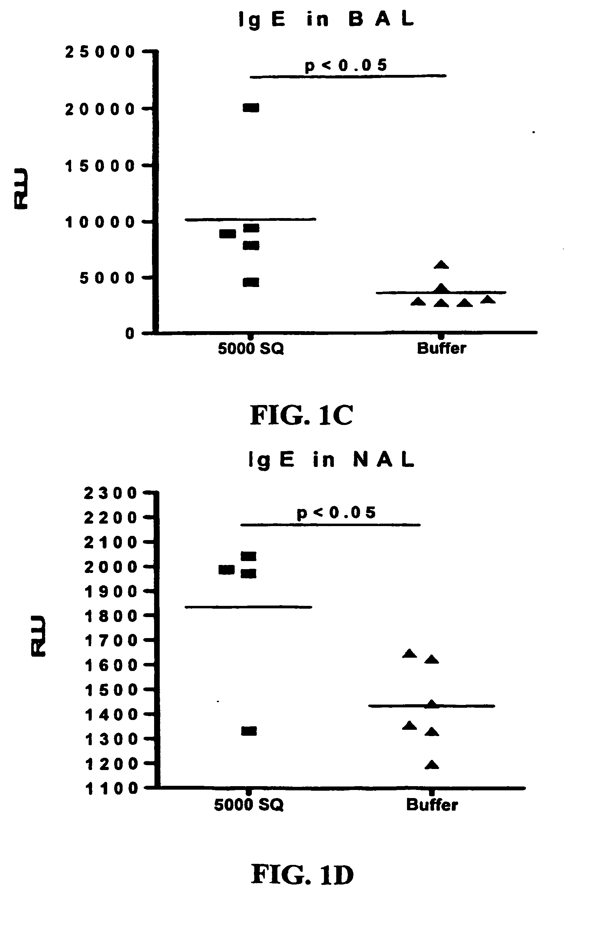

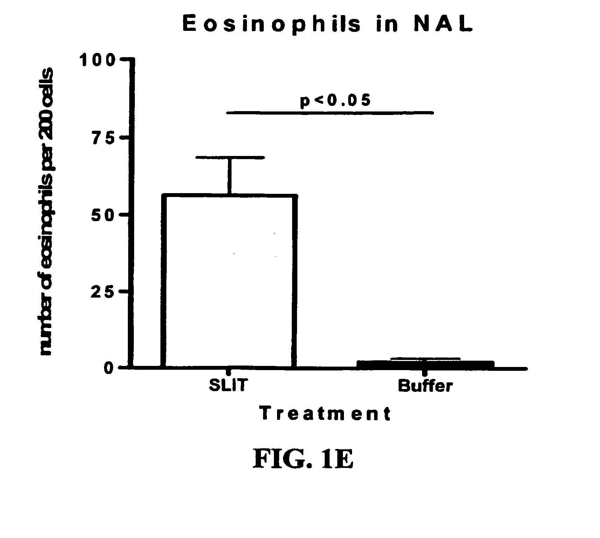

As will appear from Fig. 1A-E, mice that were challenged intranasally (IN) for two weeks with Phl p extract (5000 SQ/mouse per day) after i.p. sensitization displayed clear signs of rhinitis. Firstly, they sneezed significantly more than sensitized mice challenged with buffer (Fig. 1A). Secondly, they had elevated levels of IgE in both serum, BAL and NAL (Fig. 1B-D). Thirdly, these mice had an increased influx of eosinophil granulocytes in NAL (Fig. 1E).

### The effect of SLIT in a mouse model of rhinitis

In order to assess the effect of SLIT in the above-described murine model of rhinitis, sensitized mice were treated sublingually for 9 weeks with either 125.000 SQ Phl p extract or buffer prior to intranasal challenge with Phl p. In addition a group of sensitised mice were not subjected to sublingual treatment, and they were challenged intranasally with buffer thus serving as a negative control.

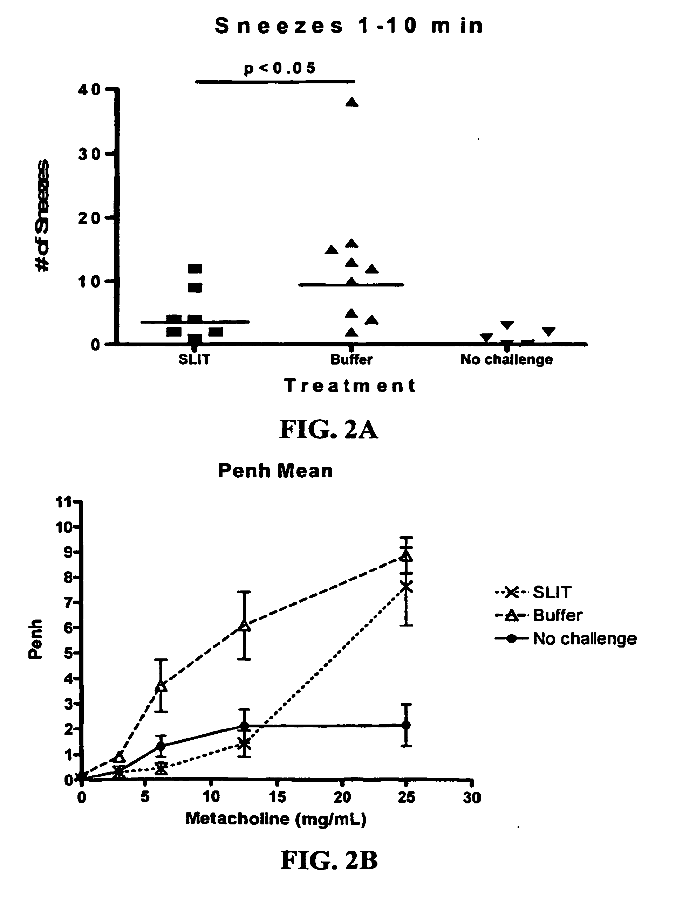

As shown in Fig. 2, SLIT-treatment was able to reduce clinical symptoms, as the number of sneezes was decreased significantly compared to buffer-treated mice (Fig. 2A). Similarly, airway hyperresponsiveness in response to metacholine challenge (as measured by Penh values) was reduced, especially when the mice were challenged with doses between 5 and 15 mg/mL (Fig. 2B).

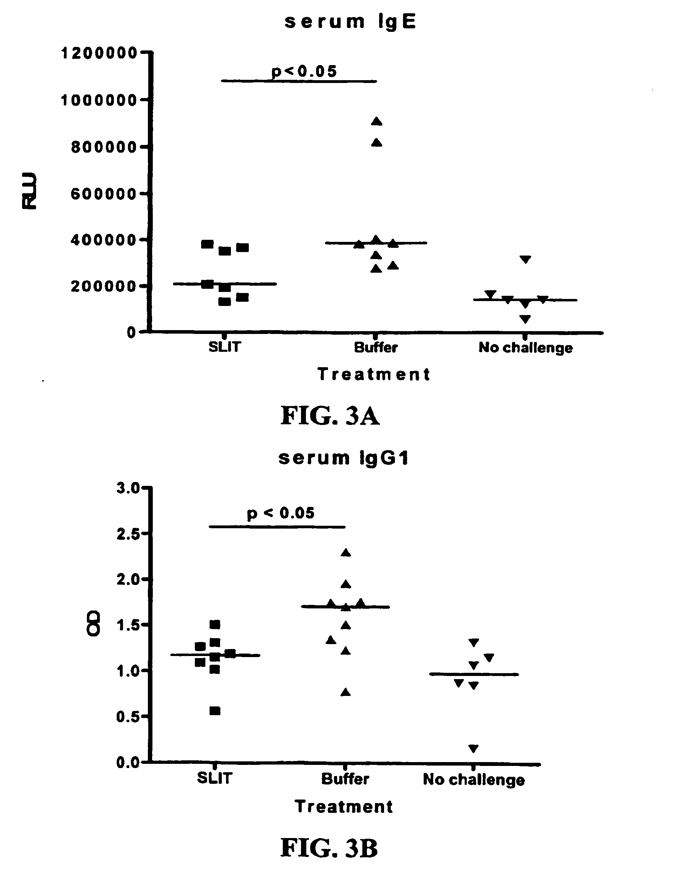

Also, antibody levels in serum were affected by the SLIT-treatment. As seen in Fig. 3, the serum levels of both Phl p specific IgE (Fig. 3A) and IgG1 (Fig. 3B) was significantly reduced in SLIT-treated mice compared to buffer treated mice.

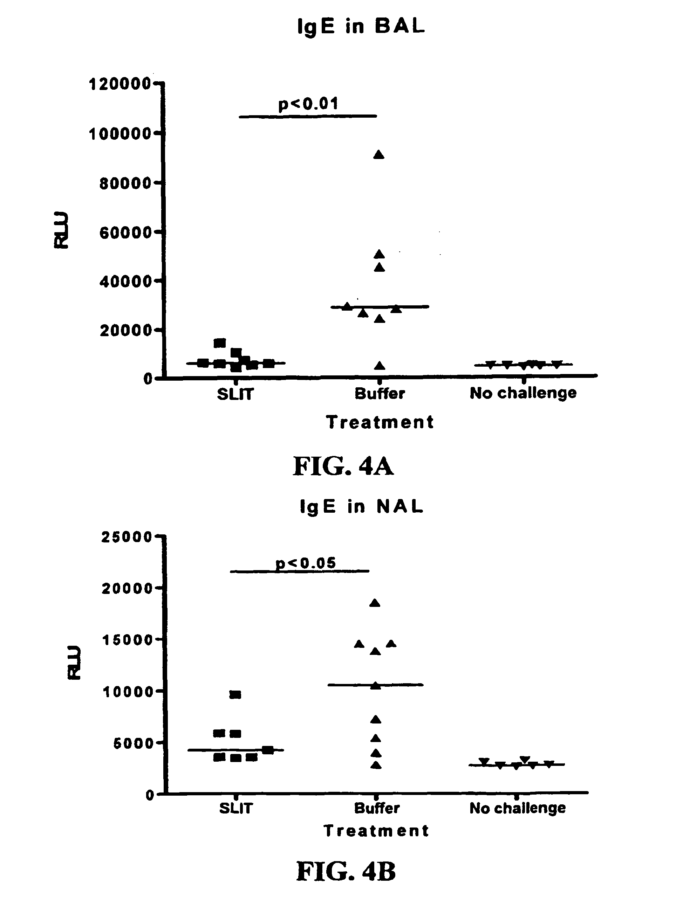

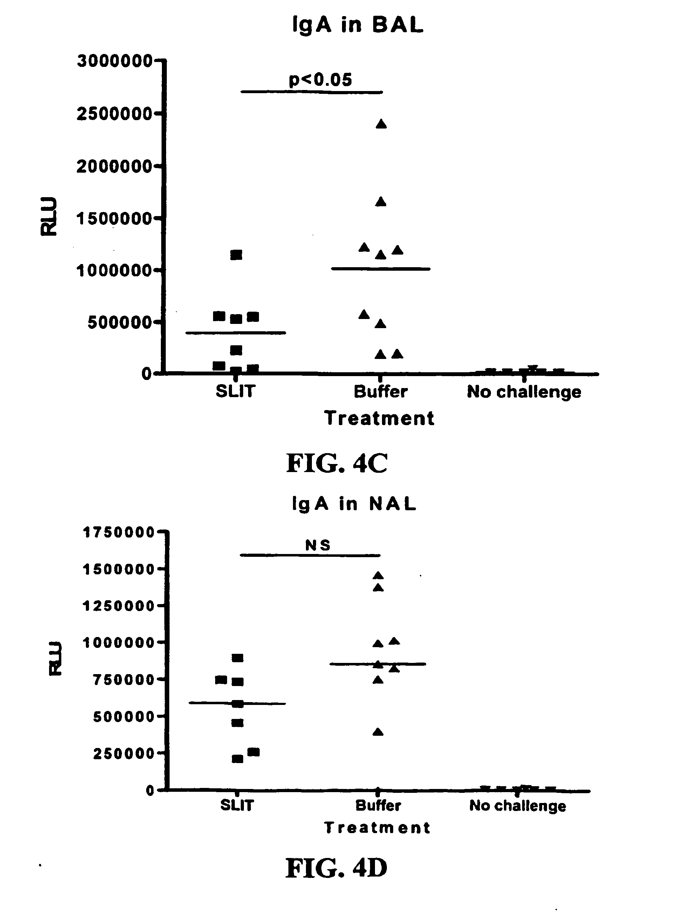

The levels of specific IgE were also decreased in both BAL and NAL fluids of SLIT-treated mice, whereas the levels of specific IgA was reduced in BAL but not significantly in NAL fluids (see Fig. 4A-C).

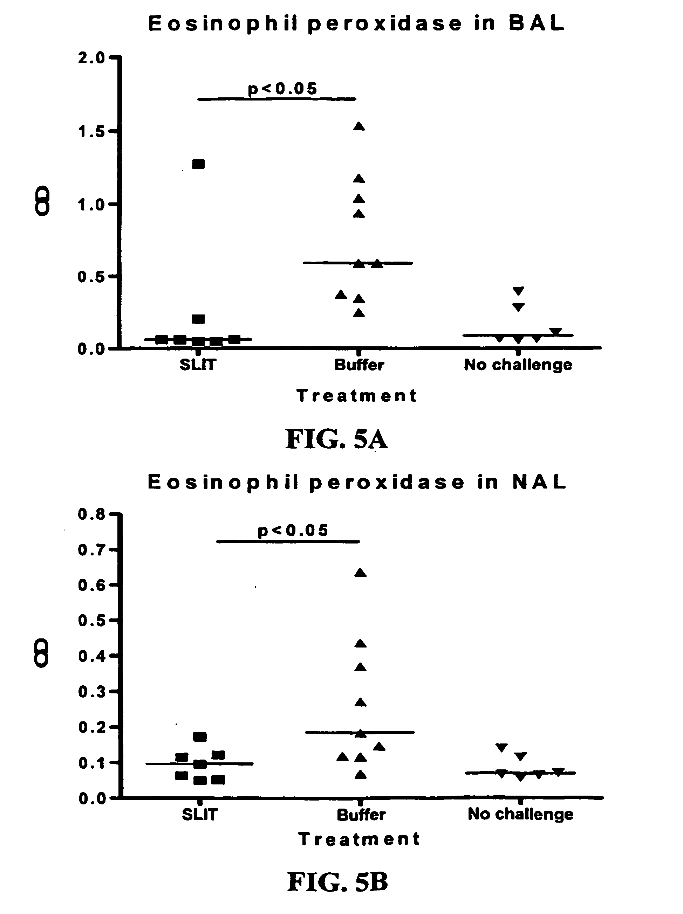

Furthermore, the results indicate that the levels of eosinophil granulocytes in both BAL and NAL were significantly reduced in SLIT-treated mice compared to buffer treated mice (see Fig. 5A-B).

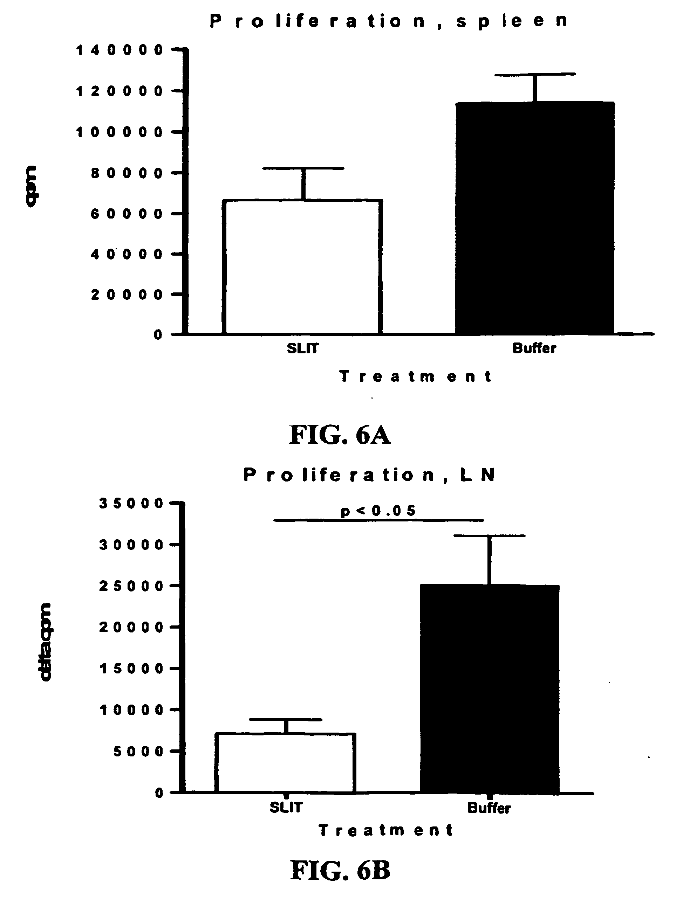

Finally, the ex vivo reactivity of spleen and cervical lymph node (LN) cells (draining the tounge) upon re-stimulation with Phl p extract was examined. Although there was a trend towards downregulation of the Phl p-specific T-cell response in the spleen, this was not statistically significant (Fig. 6A). In contrast to this, the T-cell response towards Phl p in draining cervical LNs (Fig. 6B) was significantly decreased in SLIT-treated mice compared to buffer-treated mice.

### Conclusion

The results demonstrate that SLIT treatment is able to reduce both the clinical, serological and cellular features of disease in a murine model of rhinitis.

## Claims

1. An allergen for use in preventive treatment of allergy by administering an allergy vaccine comprising the allergen to a mucosal surface of a subject,
a) wherein the subject to be treated is sensitised so as to exhibit an IgE response specific to the allergen,
b) wherein the subject to be treated is free of clinical symptoms of the allergy associated with the allergen, and
c) wherein the preventive treatment is aimed at preventing or reducing subsequent clinical symptoms of the allergy associated with the allergen.

2. The allergen according to claim 1, wherein the subject is sensitised to exhibit a Th2 cell response specific to the allergen.

3. The allergen according to claim 1 or 2, wherein the subject is free of the clinical symptoms of rhinitis, conjunctivitis, rhinorrhea, nasal obstruction, sinusitis, sneezing, atopic dermatitis, itching, watery eyes, watery nose, wheezing and skin irritation.

4. The allergen according to any of claims 1-3, wherein the subject is less than 40 years, preferably less than 30 years, more preferably less than 20 years and most preferably between 2 and 10 years of age.

5. The allergen according to any of claims 1-4, which is selected from the group consisting of an inhalation allergen and a venom allergen.

6. The allergen according to claim 5, which is selected from the group consisting of a tree pollen allergen, a grass pollen allergen, a dust mite allergen, a herb allergen and an animal allergen.

7. The allergen according to any of claims 1-6, wherein the administration is oral via the mucosa of the digestive system, nasal, vaginal, sublingual, ocular, rectal, urinal, intramammal, pulmonal, otolar or buccal administration

8. The allergen according to claim 7, wherein the administration is buccal or sublingual administration.

9. The allergen according to any of the preceding claims, wherein the subject to be treated has clinical symptoms of allergies associated with one or more allergens other than the allergen of the vaccine.

10. Use of an allergen in the preparation of an allergy vaccine comprising the allergen as active substance for preventive treatment of allergy by administering the allergy vaccine to a mucosal surface of a subject,
a) wherein the subject to be treated is sensitised so as to exhibit an IgE response specific to the allergen,
b) wherein the subject to be treated is free of clinical symptoms of the allergy associated with the allergen, and
c) wherein the preventive treatment is aimed at preventing or reducing subsequent clinical symptoms of the allergy associated with the allergen.

11. The use according to claim 10, wherein the subject is sensitised to exhibit a Th2 cell response specific to the allergen.

12. The use according to claim 10 or 11, wherein the subject is free of the clinical symptoms of rhinitis, conjunctivitis, rhinorrhea, nasal obstruction, sinusitis, sneezing, atopic dermatitis, itching, watery eyes, watery nose, wheezing and skin irritation.

13. The use according to any of claims 10-12, wherein the subject is less than 40 years, preferably less than 30 years, more preferably less than 20 years and most preferably between 2 and 10 years of age.

14. The use according to any of claims 10-13, wherein the allergen is selected from the group consisting of an inhalation allergen and a venom allergen.

15. The use according to claim 14, wherein the allergen is selected from the group consisting of a tree pollen allergen, a grass pollen allergen, a dust mite allergen, a herb allergen and an animal allergen.

16. The use according to any of claims 10-15, wherein the administration is oral via the mucosa of the digestive system, nasal, vaginal, sublingual, ocular, rectal, urinal, intramammal, pulmonal, otolar or buccal administration

17. The use according to claim 16, wherein the administration is buccal or sublingual administration.

18. The use according to any of claims 10-17, wherein the subject to be treated has clinical symptoms of allergies associated with one or more allergens other than the allergen of the vaccine.

## Patentansprüche

1. Allergen zur Verwendung für vorbeugende Behandlung von Allergie durch Verabreichung einer Allergie-Vakzine, die das Allergen umfasst, an eine Schleimhautoberfläche eines Patienten,
a) wobei der zu behandelnde Patient sensibilisiert wird, um eine allergenspezifische IgE-Antwort aufzuweisen,
b) wobei der zu behandelnde Patient frei von klinischen Symptomen der Allergie ist, welche mit dem Allergen verbunden sind, und
c) wobei die vorbeugende Behandlung die späteren klinischen Symptome der Allergie, die mit dem Allergen verbunden sind, verhindern oder reduzieren soll.

2. Allergen nach Anspruch 1, wobei der Patient sensibilisiert wird eine allergenspezifische Th2-Zellantwort zu zeigen.

3. Allergen nach Anspruch 1 oder 2, wobei der Patient frei von klinischen Symptomen von Rhinitis, Bindehautentzündung, Rhinorrhoe, Nasenverstopfung, Sinusitis, Niesen, atopischer Dermatitis, Juckreiz, Augentränen, Triefnase, Keuchen und Hautreizung ist.

4. Allergen nach einem der Ansprüche 1-3, wobei der Patient jünger als 40 Jahre alt ist, vorzugsweise jünger als 30 Jahre alt ist, noch mehr bevorzugt jünger als 20 Jahre alt ist und am stärksten bevorzugt zwischen 2 und 10 Jahren alt ist.

5. Allergen nach einem der Ansprüche 1-4, welches ausgewählt wird aus der Gruppe bestehend aus einem Inhalationsallergen und einem Giftallergen.

6. Allergen nach Anspruch 5, welches ausgewählt wird aus der Gruppe bestehend aus einem Baumpollenallergen, einem Grasspollenallergen, einem Hausstaubmilbenallergen, einem Kräuterallergen und einem Tierallergen.

7. Allergen nach einem der Ansprüche 1-6, wobei die Verabreichung oral über die Schleimhaut des Verdauungssystems, nasal, vaginal, sublingual, okular, rektal, urinal, intramammär, pulmonal, otolar oder buccal erfolgt.

8. Allergen nach Anspruch 7, wobei die Verabreichung eine buccale oder sublinguale Verabreichung ist.

9. Allergen nach einem der vorangehenden Ansprüche, wobei der zu behandelnde Patient klinische Symptome von Allergien aufweist, welche mit einem oder mehreren Allergenen, ausser dem Allergen der Vakzine, verbunden sind.

10. Verwendung eines Allergens zur Herstellung einer Allergie-Vakzine, welche das Allergen zur vorbeugenden Allergie-Behandlung als aktive Substanz umfasst, durch Verabreichen der Allergie-Vakzine an eine Schleimhautoberfläche eines Patienten,
a) wobei der zu behandelnde Patient sensibilisiert wird, um eine allergenspezifische IgE-Antwort aufzuweisen,
b) wobei der zu behandelnde Patient frei von klinischen Symptomen der Allergie ist, welche mit dem Allergen verbunden sind, und
c) wobei die vorbeugende Behandlung die späteren klinischen Symptome der Allergie, die mit dem Allergen verbunden sind, verhindern oder reduzieren soll.

11. Verwendung nach Anspruch 10, wobei der Patient sensibilisiert wird eine allergenspezifische Th2-Zellantwort zu zeigen.

12. Verwendung nach Anspruch 10 oder 11, wobei der Patient frei von klinischen Symptomen von Rhinitis, Bindehautentzündung, Rhinorrhoe, Nasenverstopfung, Sinusitis, Niesen, atopischer Dermatitis, Juckreiz, Augentränen, Triefnase, Keuchen und Hautreizung ist.

13. Verwendung nach einem der Ansprüche 10-12, wobei der Patient jünger als 40 Jahre alt ist, vorzugsweise jünger als 30 Jahre alt ist, noch mehr bevorzugt jünger als 20 Jahre alt ist und am stärksten bevorzugt zwischen 2 und 10 Jahren alt ist.

14. Verwendung nach einem der Ansprüche 10-13, wobei das Allergen ausgewählt wird aus der Gruppe bestehend aus einem Inhalationsallergen und einem Giftallergen.

15. Verwendung nach Anspruch 14, wobei das Allergen ausgewählt wird aus der Gruppe bestehend aus einem Baumpollenallergen, einem Grasspollenallergen, einem Hausstaubmilbenallergen, einem Kräuterallergen und einem Tierallergen.

16. Verwendung nach einem der Ansprüche 10-15, wobei die Verabreichung oral über die Schleimhaut des Verdauungssystems, nasal, vaginal, sublingual, okular, rektal, urinal, intramammär, pulmonal, otolar oder buccal erfolgt.

17. Verwendung nach Anspruch 16, wobei die Verabreichung eine buccale oder sublinguale Verabreichung ist.

18. Verwendung nach einem der Ansprüche 10-17, wobei der zu behandelnde Patient klinische Symptome von Allergien aufweist, welche mit einem oder mehreren Allergenen, ausser dem Allergen der Vakzine, verbunden sind.

## Revendications

1. Un allergène destiné à une utilisation dans un traitement préventif d'une allergie par administration d'un vaccin contre l'allergie comprenant l'allergène à la surface d'une muqueuse d'un sujet,
a) dans lequel le sujet à traiter est sensibilisé de façon à présenter une réponse IgE spécifique de l'allergène,
b) dans lequel le sujet à traiter ne présente pas les symptômes cliniques de l'allergie associée à l'allergène, et
c) dans lequel le traitement préventif vise à prévenir ou réduire les symptômes cliniques consécutifs à l'allergie associée à l'allergène.

2. L'allergène selon la revendication 1, dans lequel le sujet est sensibilisé pour présenter une réponse de la cellule Th2 spécifique de l'allergène.

3. L'allergène selon la revendication 1 ou 2, dans lequel le sujet ne présente pas les symptômes cliniques de la rhinite, conjonctivite, rhinorrhée, d'une obstruction nasale, d'une sinusite, d'un éternuement, d'une dermatite atopique, d'un prurit, des yeux aqueux, d'un nez coulant, de cornage ou d'une irritation de la peau.

4. L'allergène selon l'une des revendications 1 à 3, dans lequel le sujet a moins de 40 ans, de préférence moins de 30 ans, plus préférentiellement moins de 20 ans et idéalement entre 2 et 10 ans.

5. L'allergène selon l'une des revendications 1 à 4, qui est choisi dans le groupe constitué par un allergène par inhalation et un allergène de venin.

6. L'allergène selon la revendication 5, qui est choisi dans le groupe constitué par un allergène de pollen d'arbre, un allergène de pollen de graminée, un allergène d'acarien de la poussière, un allergène d'herbe et un allergène d'animal.

7. L'allergène selon l'une des revendications 1 à 6, dans lequel l'administration est orale *via* la muqueuse du système digestif, nasale, vaginale, sublinguale, oculaire, rectale, urinaire, intramammaire, pulmonaire, otorhinolaryngologique ou est une administration buccale.

8. L'allergène selon la revendication 7, dans lequel l'administration est une administration buccale ou sublinguale.

9. L'allergène selon l'une des revendications précédentes, dans lequel le sujet à traiter présente les symptômes cliniques des allergies associées à un ou plusieurs allergènes autres que l'allergène du vaccin.

10. Utilisation d'un allergène dans la préparation d'un vaccin contre une allergie comprenant l'allergène en tant que principe actif, pour un traitement préventif de l'allergie par administration du vaccin contre l'allergie à la surface d'une muqueuse d'un sujet,
a) dans laquelle le sujet à traiter est sensibilisé de façon à présenter une réponse IgE spécifique de l'allergène,
b) dans laquelle le sujet à traiter ne présente pas les symptômes cliniques de l'allergie associée à l'allergène, et
c) dans laquelle le traitement préventif vise à prévenir ou réduire les symptômes cliniques consécutifs à l'allergie associée à l'allergène.

11. L'utilisation selon la revendication 10, dans lequel le sujet est sensibilisé pour présenter une réponse de la cellule Th2 spécifique de l'allergène.

12. L'utilisation selon la revendication 10 ou 11, dans laquelle le sujet ne présente pas les symptômes cliniques de la rhinite, conjonctivite, rhinorrhée, d'une obstruction nasale, d'une sinusite, d'un éternuement, d'une dermatite atopique, d'un prurit, des yeux aqueux, d'un nez coulant, de cornage ou d'une irritation de la peau.

13. L'utilisation selon l'une des revendications 10 à 12, dans laquelle le sujet a moins de 40 ans, de préférence moins de 30 ans, plus préférentiellement moins de 20 ans et idéalement entre 2 et 10 ans.

14. L'utilisation selon l'une des revendications 10 à 13, dans laquelle l'allergène est choisi dans le groupe constitué par un allergène par inhalation et un allergène de venin.

15. L'utilisation selon la revendication 14, dans laquelle l'allergène est choisi dans le groupe constitué par un allergène de pollen d'arbre, un allergène de pollen de graminée, un allergène d'acarien de la poussière, un allergène d'herbe et un allergène d'animal.

16. L'utilisation selon l'une des revendications 10 à 15, dans laquelle l'administration est orale *via* la muqueuse du système digestif, nasale, vaginale, sublinguale, oculaire, rectale, urinaire, intramammaire, pulmonaire, otorhinolaryngologique ou est une administration buccale.

17. L'utilisation selon la revendication 16, dans laquelle l'administration est une administration buccale ou sublinguale.

18. L'utilisation selon l'une des revendications 10 à 17, dans laquelle le sujet à traiter présente les symptômes cliniques d'allergies associées à un ou plusieurs allergènes autres que l'allergène du vaccin.
